# EUROPEAN PATENT APPLICATION

(11) **EP 2 990 402 A1**
(43) Date of publication of application: **02.03.2016**
(21) Application number: 15190570.0
(22) Date of filing: 08.08.2006
(51) Int. Cl.: C07D 209/12, C07D 491/04, C07D 231/12

(54) **POSITIVELY CHARGED WATER-SOLUBLE PRODRUGS OF ARYL- AND HETEROARYLACETIC ACIDS WITH VERY FAST SKIN PENETRATION RATE**

(62) Divisional of application: 06795612.8
(71) Applicant: Techfields Biochem Co. Ltd, Shanghai N/A 200444 (CN); Yu, Chongxi, Plainfield, Illinois 60585 (US)
(72) Inventor: YU, Chongxi, Plainfield, IL Illinois 60585 (US); XU, Lina, 200444 Shanghai N/A (CN)
(74) Representative: Viering, Jentschura & Partner mbB Patent- und Rechtsanwälte

(57) **Abstract**

The novel positively charged pro-drugs of aryl- and heteroarylacetic acids in the general formula(1) 'Structure 1' were designed and synthesized. The compounds of the general formula(1) 'Structure 1' indicated above can be prepared from functional derivatives of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, (for example acid halides or mixed anhydrides), by reaction with suitable alcohols, thiols, or amines. The positively charged amino groups of these pro-drugs not only largely increases the solubility of the drugs, but also bonds to the negative charge on the phosphate head group of membranes and pushes the pro-drug into the cytosol. The results suggest that the pro-drugs diffuses through human skin ∼100 times faster than does tolmetin, zomepirac, etodolac, amfenac, bromofenac, alclofenac, fenclofenac, acemetacin, indomethacin, sulindac, fentiazac, lonazolac, bendazac, or related compounds. It takes 2-4 hours for tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds to reach the peak plasma level when they are taken orally, but these prodrugs only took about 40-50 minutes to reach the peak plasma level when they are taken transdermally. In plasma, more than 90% of these pro-drugs can change back to the drug in a few minutes. The prodrugs can be used medicinally in treating any NSAIAs-treatable conditions in humans or animals. The prodrugs can be administered not only orally, but also transdermally for any kind of medical treatments and avoid most of the side effects of NSAIAs, most notably GI disturbances such as dyspepsia, gastroduodenal bleeding, gastric ulcerations, and gastritis. Controlled transdermal administration systems of the prodrug enables tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds to reach constantly optimal therapeutic blood levels to increase effectiveness and reduce the side effects of NSAIAs. Another great benefit of transdermal administration of these pro-drugs is that administering medication, especially to children, will be much easier.

## Description

### Technical Field

The present invention relates to the preparations of positively charged and watersoluble pro-drugs of aryl- and heteroarylacetic acids and their medicinal use in treating any nonsteroidal anti-inflammatory drugs (NSAIAs)-treatable conditions in humans or animals. More specifically, the present invention is to overcome the side effects that are associated with the use of NSAIAs. These pro-drugs can be administered orally or transdermally.

### Background Art

1-Methyl-5-(4-methylbenzoyl)-1H-pyrrole-2-acetic acid (tolmetin), 5-(4-Chlorobenzoyl)-1,4-dimethyl-1H-pyrrole-2-acetic acid (zomepirac), 1,8-diethyl-1,3,4,9-tetrahydropyrano-[3,4-b]indole-1-acetic acid (etodolac), 2-amino-3-benzoylbenzeneacetic acid (amfenac), 2-amino-3-(4-bromo-benzoyl) benzeneacetic acid (bromofenac), 3-chloro-4-(2-propenyloxy) benzeneacetic acid (alclofenac), 2-(2,4-dichlorophenoxy)benzeneacetic acid (fenclofenac), 1-(4-chlorobenzoyl-5-methoxy-2-methyl-1H-indole-3-acetic acid carboxymethyl ester (acemetacin), 4-(4-chlorophenyl)-2-phenyl-5-thiazoleacetic acid (fentiazac), 1-(4-chlorobenzoyl)-5-methoxy-2-methyl-1H-indole-3-acetic acid (indomethacin), 5-fluoro-2-methyl-1-[[(4-methylsulfinyl) phenyl]methylene]-1H-indene-3-acetic acid (sulindac), 3-(4-chlorophenyl)-1-phenyl-1H-pyrazole-4-acetic acid (lonazolac), [(1-benzyl-1H-indazol-3-yl)oxy]acetic acid (bendazac), 6-methoxyl-2-naphthalene-2-acetic acid (6MNA), p-isobutylphenylacetic acid (ibufenac), and related compounds are members of aryl- and heteroarylacetic acid group of nonsteroidal anti-inflammatory drugs. They are used for the relief of signs and symptoms of rheumatoid arthritis and osteoarthritis and for the treatment of dysmenorrheal. They are also used for the treatment of acute gouty arthritis and ankylosing spondylitis. The may be used for the treatment of dementia (McGeer; Patrick L. et al. U.S. Pat. No.5,192,753 ).

Unfortunately, a number of side effects are associated with the use of tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the blood stream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Roentsch, et al., U.S. Pat. No. 5,654,337, Park, et al., U.S. Pat. No. 6,190,690, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of these kind drugs into the host by formulation, due to the slow skin penetration rate. Susan Milosovich, et. al. designed and prepared testosteronyl-4-dimethylaminobutyrate.HCl (TSBH), which has a lipophilic portion and a tertiary amine groups that exists in the protonated form at physiological pH. They found that the prodrug (TSBH) diffuses through human skin -60 times faster than does the drug (TS) itself [Susan Milosovich, et al., J. Pharm. Sci., 82, 227(1993).

### Disclosure of Invention

### Technical Problem

Tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds, have been used medicinally for many years. They are used for the relief of signs and symptoms of rheumatoid arthritis and osteoarthritis, for the treatment of dysmenorrhea.

Unfortunately, a number of side effects are associated with the use of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, most notably GI disturbances such as dyspepsia, gastroduodenal bleeding, gastric ulcerations, and gastritis. They are not soluble in aqueous solution and gastric juice. They stay in the GI tract for a long time and thus, may cause gastric mucosal cell damage.

### Technical Solution

This invention relates to the preparation of novel positively charged pro-drugs of tolmetin, zomepirac, etodolac, amfenac, bromofenac, indomethacin, sulindac, alclofenac, fenclofenac, acemetacin, fentiazac, lonazolac, bendazac, 6MNA, ibufenac, and related compounds and their use medicinally. These pro-drugs have the general formula (1) 'Structure 1'. 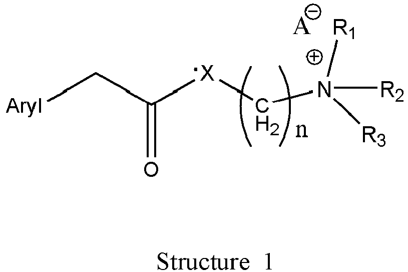 In structure 1, R₁ represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S, NH, OCH₂COO, OCH₂COS, or OCH₂CONH,; A⁻ represents Cl⁻, Br⁻, F⁻, I⁻, AcO⁻, citrate, or any negative ions; and n=0,1,2,3,4,5,6,7,8,9,10 ......; Aryl represents 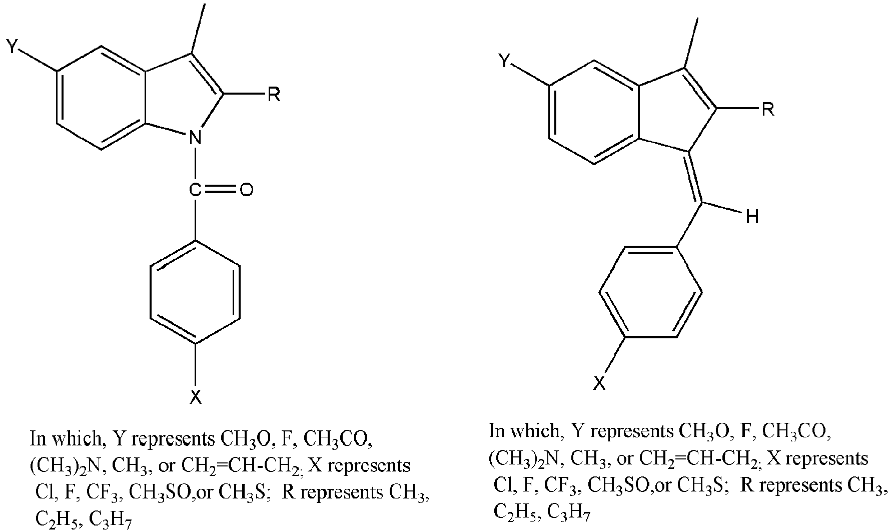 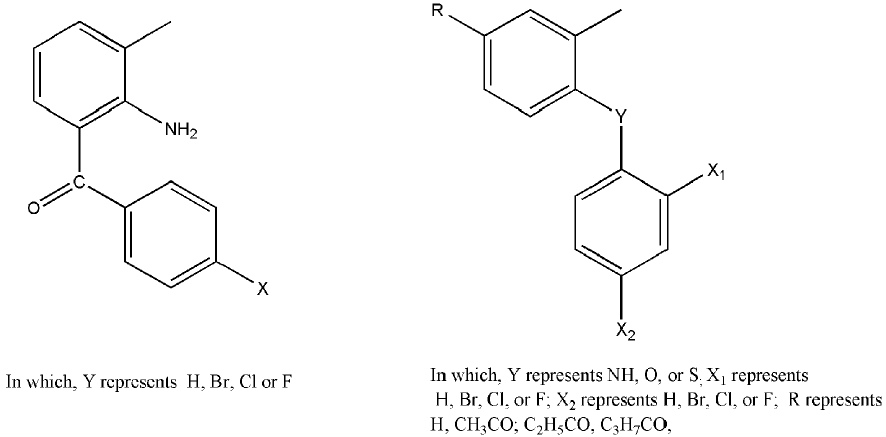 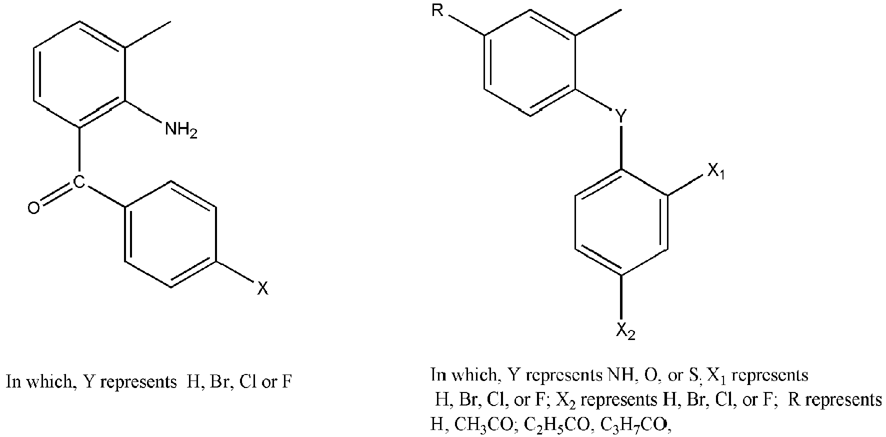 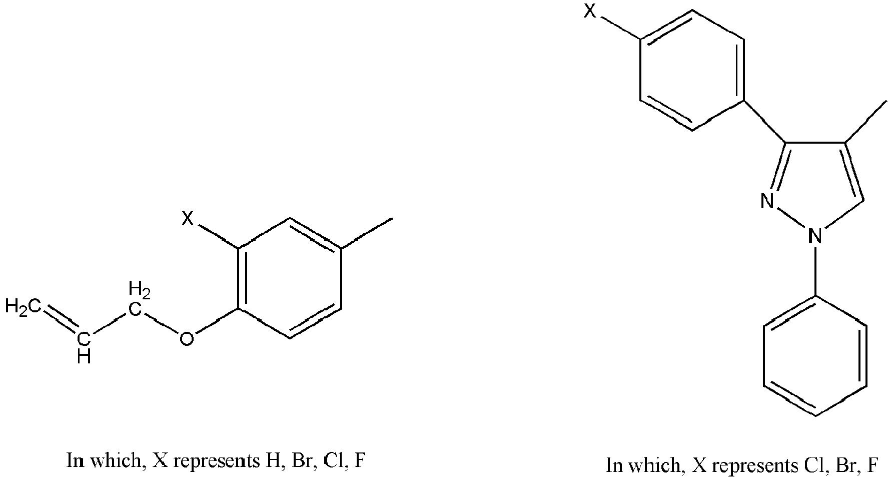 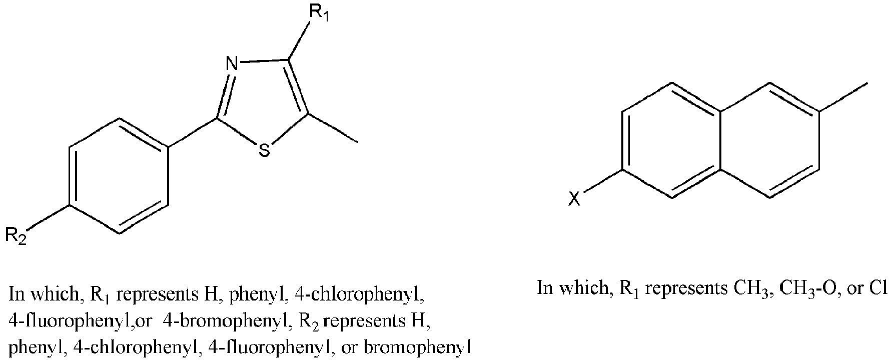 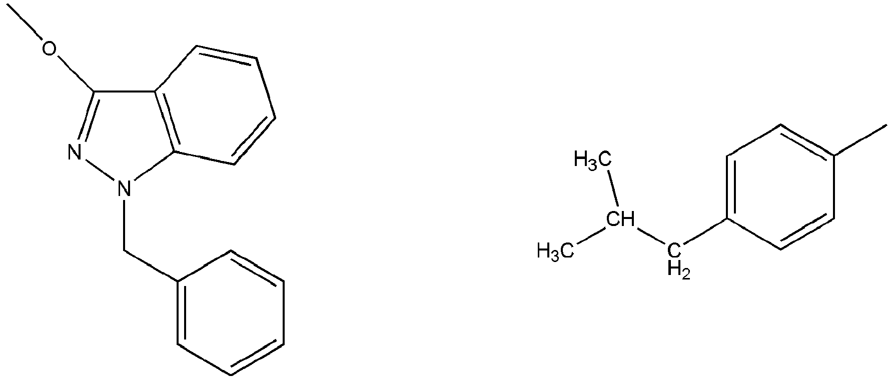 , All R groups may include C, H, O, S, N atoms and may have single, double, and treble bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

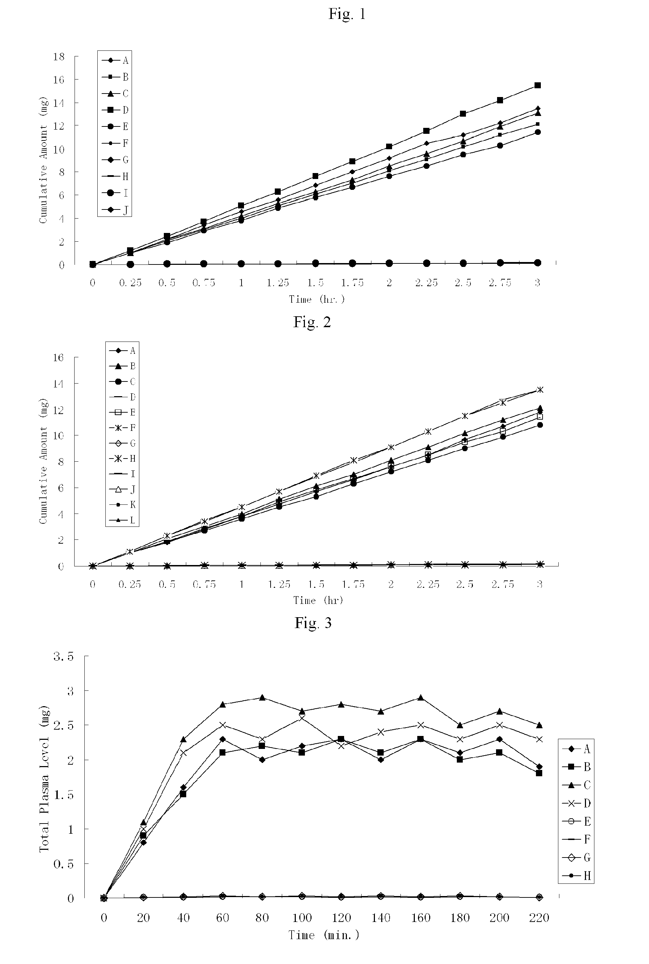

The goal of this invention is to avoid the side effects of tolmetin, zomepirac, etodolac, indomethacin, sulindac, amfenac, bromofenac, alclofenac, fenclofenac, acemetacin, fentiazac, lonazolac, bendazac, 6MNA, ibufenac, and related compounds by increasing the their solubility in gastric juice and their penetration rate through the membrane and skin barrier which will make it administrable transdermally (topical application). These novel pro-drugs have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH, diethylaminoethyl [(1-benzyl-1H-indazol-3-yl)oxy]acetate.AcOH, diethylaminoethyl 6-methoxyl-2-naphthalene-2-acetate.AcOH, diethylaminoethyl p-isobutylphenylacetate.AcOH, 1-(p-chlorobenzoyl)-5-methoxy-2-methylindole 3-acetic acid (indomethacin), (Z)-5-fluoro-2-methyl-1-[(4-methylsulfinyl) phenylmethylene] - 1H-indene-3-acetic acid (sulindac), 1-methyl-5-(4-methylbenzoyl)-1H-pyrrole-2-acetic acid (tolmetin), 5-(4-Chlorobenzoyl)-1,4-dimethyl-1H-pyrrole-2-acetic acid (zomepirac), 1,8-diethyl-1,3,4,9-tetrahydropyrano-[3,4-b]indole-1-acetic acid (etodolac), 2-amino-3-benzoylbenzeneacetic acid (amfenac), 2-amino-3-(4-bromo-benzoyl)benzeneacetic acid (bromofenac), 3-chloro-4-(2-propenyloxy)benzeneacetic acid (alclofenac), 2-(2,4-dichlorophenoxy)benzeneacetic acid (fenclofenac), 1-(4-chlorobenzoyl-5-methoxy-2-methyl-1H-indole-3-acetic acid carboxymethyl ester (acemetacin), 4-(4-chlorophenyl)-2-phenyl-5-thiazoleacetic acid (fentiazac), or 3-(4-chlorophenyl)-1-phenyl-1H-pyrazole-4-acetic acid (lonazolac), [(1-benzyl-1H-indazol-3-yl)oxy]acetic acid (bendazac), 6-methoxyl-2-naphthalene-2-acetic acid (6MNA), p-isobutylphenylacetic acid (ibufenac) in water are >450 mg, >400 mg, >450 mg, >450 mg, >350 mg, >450 mg, > 400 mg, >450 mg, >400 mg, >450 mg, >350 mg, >400 mg, >350 mg, >400 mg, >350 mg, 0.1 mg, 0.1 mg, 0.2 mg, 0.2 mg, 0.1 mg, 0.2 mg, 0.1 mg, 0.1 mg, 0.1 mg, 0.1 mg, 0.1 mg, 0.1 mg/ml, 0.1 mg, 0.1 mg, and 0.1 mg/ml. In many instances, the lowest or rate-limiting step in the sequence is the dissolution of the drug. Tolmetin, zomepirac, etodolac, amfenac, bromofenac, alclofenac, fenclofenac, indomethacin, sulindac, acemetacin, fentiazac, lonazolac, bendazac, 6MNA, ibufenac, and related compounds have a very low solubility in gastric juice. They stay in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the prodrugs will not cause gastric mucosal cell damage. The penetration rates of diethylaminoethyl 1-(p-chlorobenzoyl)-5-methoxy-2-methylindole 3-acetate.AcOH, diethylaminoethyl 5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, [(1-benzyl-1H-indazol-3-yl)oxy]acetate.AcOH, 3-(4-chlorophenyl)-1-phenyl-1H-pyrazole-4-acetate.AcOH, [(1-benzyl-1H-indazol-3-yl)oxy]acetate.AcOH, indomethacin, sulindac, tolmetin, zomepirac, etodolac, amfenac, bromofenac, alclofenac, fenclofenac, acemetacin, fentiazac, lonazolac, bendazac, and related compounds through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 30% solution of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, or a 30% suspension of indomethacin, sulindac, tolmetin, zomepirac, etodolac, amfenac, bromofenac, alclofenac, fenclofenac, acemetacin, or fentiazac in 2mL of pH 7.4 phosphate buffer (0.2M) are shown in Figure 1 or Figure 2. Apparent flux values of 4.6 mg, 4 mg, 4.2 mg, 5.1 mg, 4.2 mg, 3.8 mg, 4.0 mg, 3.6 mg, 4.5 mg, 3.8 mg, 4.5 mg, 0.04 mg, 0.04 mg, 0.04 mg, 0.05 mg, 0.04 mg, 0.04 mg, 0.04 mg, 0.03 mg, 0.04 mg, 0.03 mg, and 0.04 mg/cm²/h were calculated for diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, indomethacin, sulindac, tolmetin, zomepirac, etodolac, amfenac, bromofenac, alclofenac, fenclofenac, acemetacin, fentiazac, or lonazolac diffuses through human skin. The results suggest that the pro-drug, diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, or diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, or diethylaminoethyl 4-(4-chlorophenyl)-2-phenyl-5-thiazoleacetate.AcOH diffuses through human skin -100 times faster than does indomethacin, sulindac, tolmetin, zomepirac, etodolac, amfenac, bromofenac, alclofenac, fenclofenac, acemetacin, fentiazac, or lonazolac. The results suggest that the positive charge on the dialkyaminoethyl group has a very important role in the passage of the drug across the membrane and skin barrier. Other prodrugs of the general 'Structure 1' have very high penetration rates and are very close to that of diethylaminoethyl 1-(p-chlorobenzoyl)-5-methoxy-2-methylindole 3-acetate.AcOH.

The in vivo rates of penetration of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH, indomethacin, sulindac, tolmetin, zomepirac, etodolac, amfenac, bromofenac, alclofenac, fenclofenac, acemetacin, fentiazac, or lonazolac through the skin of intact hairless mice were compared. The donor consisted of a 20% solution of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, indomethacin, sulindac, tolmetin, zomepirac, etodolac, amfenac, bromofenac, alclofenac, fenclofenac, acemetacin, fentiazac, or lonazolac in 1 mL of isopropanol applied to a 10 cm² on the backs of the hairless mice. Plasma levels of indomethacin, sulindac, tolmetin, zomepirac, etodolac, amfenac, bromofenac, alclofenac, fenclofenac, acemetacin, fentiazac, or lonazolac were determined by a specific high-performance liquid chromatography method. The results (Figure 3, Figure 4, Figure 5) show that the peak levels of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, and diethylaminoethyl 3-(4-chlorophenyl)-1-phenyl-1H-pyrazole-4-acetate.AcOH were reached -50 minutes after application of the donor systems. It takes 2-4 hours for indomethacin, sulindac, tolmetin, zomepirac, etodolac, amfenac, bromofenac, alclofenac, fenclofenac, acemetacin, and fentiazac to reach their peak plasma level when they are taken orally. The peaks were -0.01 mg/ml for indomethacin, sulindac, tolmetin, zomepirac, etodolac, amfenac, bromofenac, alclofenac, fenclofenac, acemetacin, and fentiazac, lonazolac, bendazac, and -2 mg/ml for diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH, (approximately 200 times difference). -2 mg/ml of indomethacin, sulindac, tolmetin, zomepirac, etodolac, amfenac, bromofenac, alclofenac, fenclofenac, acemetacin, or fentiazac in plasma is more than 20-100 times higher than plasma level for effective analgesia and effective antiinflammatory activity. This is a very exciting result. It will be very easy and fast to deliver therapeutically effective plasma level of indomethacin, sulindac, tolmetin, zomepirac, etodolac, amfenac, bromofenac, alclofenac, fenclofenac, acemetacin, or fentiazac into the host by these pro-drugs. These results suggest that the pro-drugs can be administered not only orally, but also transdermally for any kind of medical treatments. The in vivo rates of penetration of other Pro-drugs of the general 'Structure 1' are close to that of diethylaminoethyl (Z)-5-fluoro-2-methyl-1-[(4-methylsulfinyl) phenylmethylene]-1H-indene-3-acetate.AcOH.

To check the gastroduodenal bleeding caused by drugs, rats were orally administered with 50 mg/kg of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH, indomethacin, sulindac, tolmetin, zomepirac, etodolac, amfenac, bromofenac, alclofenac, fenclofenac, acemetacin, fentiazac, or lonazolac per day for 21 days. We found an average of 3-4 mg of fecal blood per gram of feces in the indomethacin, sulindac, tolmetin, zomepirac, etodolac, amfenac, bromofenac, alclofenac, fenclofenac, acemetacin, fentiazac, or lonazolac groups and none in diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH groups.

The acute toxicity of the prodrugs was investigated. The LD₅₀ orally in rats are: 0.1 g/kg, 0.5 g/kg, 0.6g/kg , 0.2 g/kg, 0.6 g/kg , 1.0 g/kg, 1.6 g/kg, 3.0 g/kg, 0.2 g/kg, 1.2 g/kg, and 1.2 g for diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, or diethylaminoethyl 3-(4-chlorophenyl)-1-phenyl-1H-pyrazole-4-acetate.AcOH. The results show that the prodrugs are less toxic than indomethacin (LD₅₀=0.013g/kg), sulindac (LD₅₀=0.26 g/kg), tolmetin (LD₅₀=0.35 g/kg), zomepirac (LD₅₀=0.027 g/kg), etodolac (LD₅₀=0.46 g/kg), amfenac (LD₅₀=0.31 g/kg), bromofenac, alclofenac (LD₅₀ =1.05 g/ kg), fenclofenac (LD₅₀=2.28 g/kg), acemetacin (LD₅₀=0.024 g/kg), fentiazac (LD₅₀ =0.7 g/kg), or lonazolac (LD₅₀=1.0 g/kg).

Tolmetin, zomepirac, etodolac, amfenac, bromofenac, alclofenac, fenclofenac, indomethacin, sulindac, acemetacin, fentiazac, lonazolac, bendazac, 6MNA, ibufenac, and related compounds have demonstrated anti-inflammatory, analgesic, antipyretic, and antirheumatic activity. A good prodrug should go back to the drug itself in plasma. Diethylaminoethyl ester group of these prodrugs can be rapidly cleaved by the enzymes in human plasma in vitro and more than 90% of the pro-drugs are changed back to their parent drugs. Due to the pro-drugs having a much better absorption rate, the prodrugs will have more strength than their parent drugs at the same dosage. The analgetic, antipyretic, and anti-inflammatory activities of these prodrugs were tested using tolmetin, zomepirac, etodolac, amfenac, indomethacin, sulindac, bromofenac, alclofenac, fenclofenac, acemetacin, fentiazac, or lonazolac as a comparison.

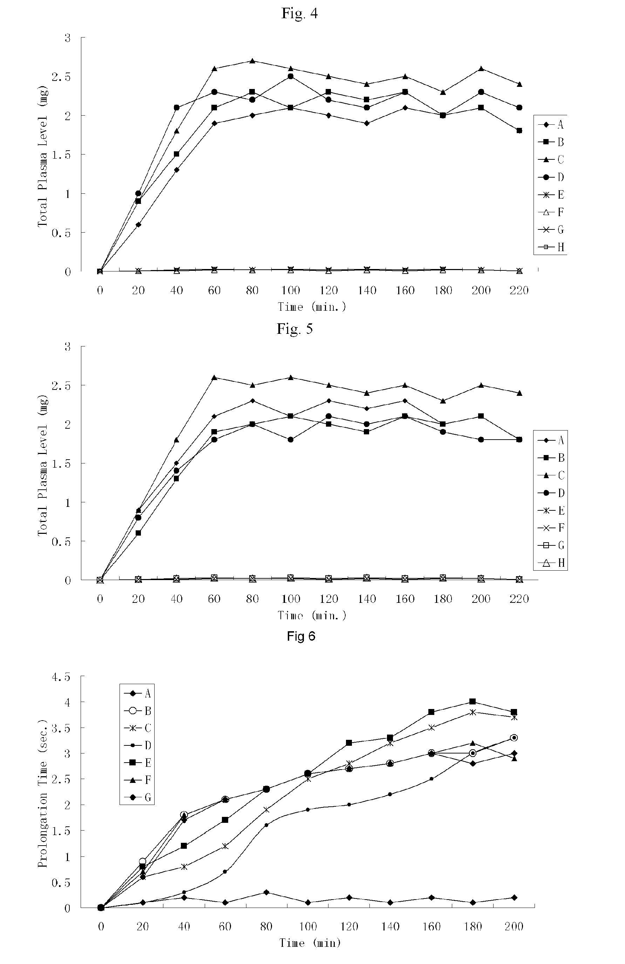

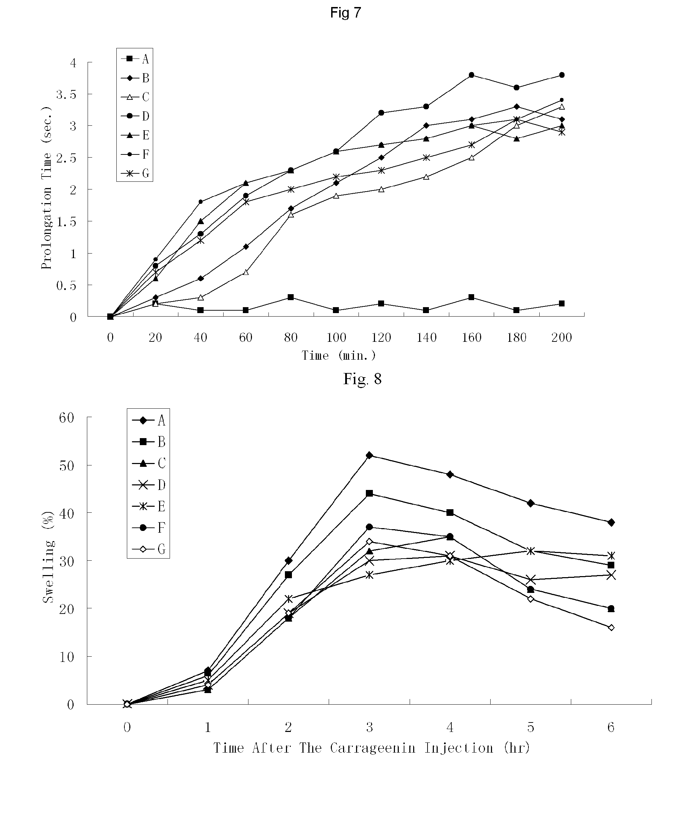

Analgetic activity: The prolongation time of pain the threshold of a mouse tail was determined in accordance with the D'Amour-Smith Method (J. Pharmacol. Exp. Ther., 72, 74(1941)). After 50 mg/kg of these prodrugs were administered transdermally, the tails of mice were exposed to heat and the prolongation time of pain threshold was determined. The results obtained are shown in Figure 6 and Figure 7. Diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH have shown analgesic activity nicely.

The number of writhings that occurred when mice were administered an acetic acid solution intraperitoneally were counted, and the rate of inhibition based on the control group was calculated. Diethylaminoethyl 1-(p-chlorobenzoyl)-5-methoxy-2-methylindole 3-acetate.AcOH (100 mg/kg, B), diethylaminoethyl (Z)-5-fluoro-2-methyl-1-[(4-methylsulfinyl) phenylmethylene] - 1H-indene-3-acetate.AcOH (100 mg/kg, C), diethylaminoethyl 1-methyl-5-(4-methylbenzoyl)-1H-pyrrole-2-acetate.AcOH (100 mg/kg, D), diethylaminoethyl 5-(4-Chlorobenzoyl)-1,4-dimethyl-1H-pyrrole-2-acetate.AcOH (100 mg/ kg, E), diethylaminoethyl 1,8-diethyl-1,3,4,9-tetrahydropyrano-[3,4-b] indole-1-acetate.AcOH (100 mg/kg, F), diethylaminoethyl 2-amino-3-benzoylbenzeneacetate.AcOH (100 mg/kg, G), diethylaminoethyl 2-amino-3-(4-bromo-benzoyl)benzeneacetate.AcOH (100 mg/kg, H), diethylaminoethyl 3-chloro-4-(2-propenyloxy)benzeneacetate.AcOH (100 mg/kg, I), diethylaminoethyl 2-(2,4-dichlorophenoxy)benzeneacetate.AcOH (100 mg/kg, J), diethylaminoethyl 1-(4-chlorobenzoyl-5-methoxy-2-methyl-1H-indole-3-acetoxyacetate.AcOH (100 mg/ kg, K), diethylaminoethyl 4-(4-chlorophenyl)-2-phenyl-5-thiazoleacetate.AcOH (100 mg/kg, L), or diethylaminoethyl 3-(4-chlorophenyl)-1-phenyl-1H-pyrazole-4-acetate.AcOH (100 mg/kg, M) were administered transdermally the mice 30 minutes before the acetic acid solution was administered. The A group is the control group. The results are shown in Table 1.

**Table 1. The rate of writhings inhibition by and ketoprofen and related compounds.**

| Group | Dose (mg/kg) | No. of Writhings | % |
|---|---|---|---|
| A | 0 | 35.0 | - |
| B | 100 | 15.6 | 55 |
| C | 100 | 14.2 | 59 |
| D | 100 | 17.1 | 51 |
| E | 100 | 15.6 | 55 |
| F | 100 | 14.0 | 60 |
| G | 100 | 13.8 | 61 |
| H | 100 | 13.2 | 62 |
| I | 100 | 15.7 | 55 |
| J | 100 | 14.2 | 59 |
| K | 100 | 15.6 | 55 |
| L | 100 | 16.1 | 54 |
| M | 100 | 15.2 | 57 |

The results show that the prodrugs demonstrate exceptional analgetic activity. Other compounds of the general 'Structure 1' show similar analgetic activity.

Antipyretic activity: Rats received a sterilized E. coli suspension as a pyrogen. The control group is group A. 2 hours later, diethylaminoethyl 1-(p-chlorobenzoyl)-5-methoxy-2-methylindole 3-acetate.AcOH (100 mg/kg, B), diethylaminoethyl (Z)-5-fluoro-2-methyl-1-[(4-methylsulfinyl) phenylmethylene] - 1H-indene-3-acetate.AcOH (100 mg/kg, C), diethylaminoethyl 1-methyl-5-(4-methylbenzoyl)-1H-pyrrole-2-acetate.AcOH (100 mg/kg, D), diethylaminoethyl 5-(4-Chlorobenzoyl)-1,4-dimethyl-1H-pyrrole-2-acetate.AcOH (100 mg/ kg, E), diethylaminoethyl 1,8-diethyl-1,3,4,9-tetrahydropyrano-[3,4-b] indole-1-acetate.AcOH (100 mg/kg, F), diethylaminoethyl 2-amino-3-benzoylbenzeneacetate.AcOH (100 mg/kg, G), diethylaminoethyl 2-amino-3-(4-bromo-benzoyl)benzeneacetate.AcOH (100 mg/kg, H), diethylaminoethyl 3-chloro-4-(2-propenyloxy)benzeneacetate.AcOH (100 mg/kg, I), diethylaminoethyl 2-(2,4-dichlorophenoxy)benzeneacetate.AcOH (100 mg/kg, J), diethylaminoethyl 1-(4-chlorobenzoyl-5-methoxy-2-methyl-1H-indole-3-acetoxyacetate.AcOH (100 mg/ kg, K), diethylaminoethyl 4-(4-chlorophenyl)-2-phenyl-5-thiazoleacetate.AcOH (100 mg/kg, L), or diethylaminoethyl 3-(4-chlorophenyl)-1-phenyl-1H-pyrazole-4-acetate.AcOH (100 mg/kg, M) were administered transdermally. The body temperature of rats was taken at 90 min. intervals before and after the administration of the test compounds. The results are shown in Table 2.

**Table 2. Antipyretic Activity of ketoprofen and related compounds.**

| Compound | t=0 min. | t=90 min. | t=180 min. | t=270 min. |
|---|---|---|---|---|
| A (Control group) | 37.33±0.05 | 37.26±0.07 | 37.32±0.05 | 37.34±0.08 |
| B (100mg/kg) | 37.35±0.06 | 36.91±0.05 | 36.85±0.08 | 36.79±0.07 |
| C (100mg/kg) | 37.28±0.06 | 36.65±0.05 | 36.62±0.08 | 36.58±0.07 |
| D (100mg/kg) | 37.27±0.06 | 36.71±0.05 | 36.65±0.08 | 36.59±0.07 |
| E (100mg/kg) | 37.21±0.07 | 36.82±0.06 | 36.70±0.05 | 36.70±0.08 |
| F (100mg/kg) | 37.23±0.06 | 36.65±0.06 | 36.58±0.08 | 36.60±0.07 |
| G (100mg/kg) | 37.22±0.06 | 36.65±0.05 | 36.62±0.08 | 36.58±0.07 |
| H (100mg/kg) | 37.25±0.06 | 36.71±0.05 | 36.65±0.08 | 36.64±0.07 |
| I (100mg/kg) | 37.23±0.07 | 36.80±0.06 | 36.70±0.05 | 36.67±0.08 |
| J (100mg/kg) | 37.22±0.06 | 36.65±0.06 | 36.58±0.08 | 36.56±0.07 |
| K (100mg/kg) | 37.21±0.06 | 36.75±0.05 | 36.62±0.08 | 36.58±0.07 |
| L (100mg/kg) | 37.23±0.06 | 36.81±0.05 | 36.75±0.08 | 36.71±0.07 |
| M (100mg/kg) | 37.22±0.07 | 36.82±0.06 | 36.80±0.05 | 36.77±0.08 |

The results shown that the prodrugs demonstrated strong antipyretic activity at 100 mg/kg dose. Other compounds of the general 'Structure 1' show similar antipyretic activity.

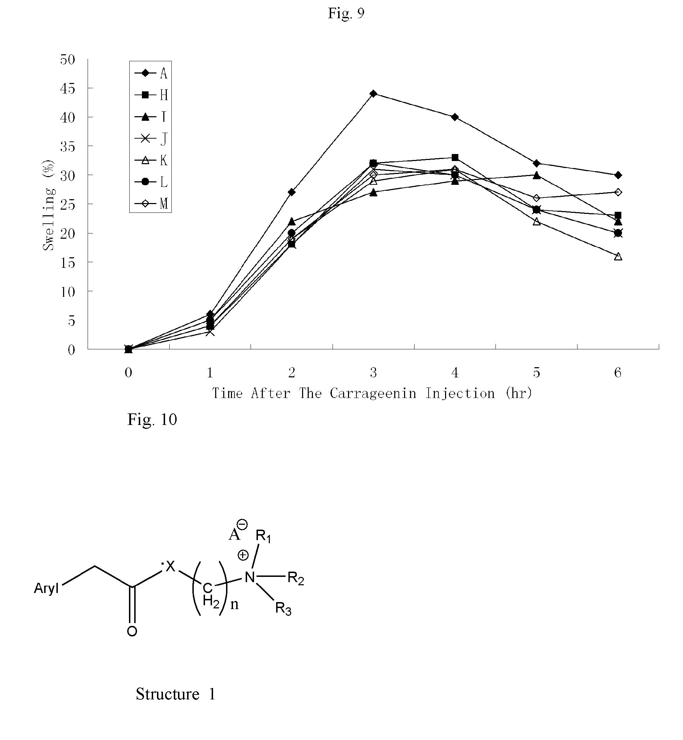

Anti-inflammatory activity: 100 mg/kg of diethylaminoethyl 1-(p-chlorobenzoyl)-5-methoxy-2-methylindole 3-acetate.AcOH (B), diethylaminoethyl (Z)-5-fluoro-2-methyl-1-[(4-methylsulfinyl) phenylmethylene] - 1H-indene-3-acetate.AcOH (C), diethylaminoethyl 1-methyl-5-(4-methylbenzoyl)-1H-pyrrole-2-acetate.AcOH (D), diethylaminoethyl 5-(4-Chlorobenzoyl)-1,4-dimethyl-1H-pyrrole-2-acetate.AcOH (E), diethylaminoethyl 1,8-diethyl-1,3,4,9-tetrahydropyrano-[3,4-b]indole-1-acetate.AcOH (F), diethylaminoethyl 2-amino-3-benzoylbenzeneacetate.AcOH (G), diethylaminoethyl 2-amino-3-(4-bromo-benzoyl)benzeneacetate.AcOH (H), diethylaminoethyl 3-chloro-4-(2-propenyloxy)benzeneacetate.AcOH (I), diethylaminoethyl 2-(2,4-dichlorophenoxy)benzeneacetate.AcOH (J), diethylaminoethyl 1-(4-chlorobenzoyl-5-methoxy-2-methyl-1H-indole-3-acetoxyacetate.AcOH (100 mg/ kg, K), diethylaminoethyl 4-(4-chlorophenyl)-2-phenyl-5-thiazoleacetate.AcOH (L), or diethylaminoethyl 3-(4-chlorophenyl)-1-phenyl-1H-pyrazole-4-acetate.AcOH (M) were administered transdermally. Group A is the controlled group. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 8 and Figure 9. The results show that these prodrugs by transdermal administration demonstrated good anti-inflammatory activity. Other compounds of the general 'Structure 1' show similar anti-inflammatory activity.

It is also known that a high oral dose of some of NSAIAs shows an anti-reactive-antiasthmatic activity by inhibition of the cyclooxygenase activity. Due to their very high membrane penetration rate, these prodrugs can be used in treating asthma by spraying into the mouth or nose of the host. They can also be used to treat acne due to their anti-inflammatory properties and very high skin penetration rate.

The present invention relates to pharmaceutical preparations comprising of prodrugs of the general 'Structure 1' in addition to customary auxiliaries and excipients, e.g. in the form of tablets, capsules or solutions for administration orally and in the form of solutions, lotion, ointment, emulsion or gel for transdermal administration. The new active compounds of the general 'Structure 1' can be combined with vitamins such as A, B, C or E or beta-carotene, or other pharmaceuticals, such as folic acid, etc., for treating any NSAIAs-treatable conditions in humans or animals.

Transdermal therapeutic application systems of compounds of the general 'Structure 1' or a composition comprising of at least one compound of the general 'Structure 1', as an active ingredient, can be used for treating any NSAIAs-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables NSAIAs to reach constantly optimal therapeutic blood levels to increase effectiveness and reduce the side effects of NSAIAs. These systems can be worn on the wrist, ankle, arm, leg, or any part of body.

The compounds of the general formula (1) 'Structure 1' indicated above can be prepared from functional derivatives of aryl- and heteroarylacetic acids, for example, acid halides or mixed anhydrides of the general formula (2) 'Structure 2'. 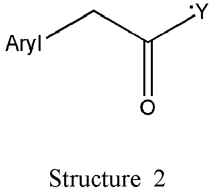 In structure 2, Y represents halogen, alkoxycarbonyl or substituted aryloxy-carbonyloxy, Aryl represents aryl- and heteroaryl groups that are listed in 'structure 1', by reaction with compounds of the general formula (3) 'Structure 3', 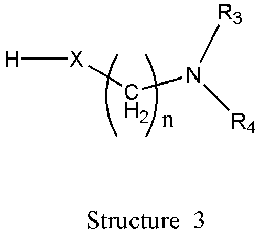 In structure 3, R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; X represents O, S or NH; and n=0,1,2,3,4,5,6,7,8,9,10.......

The compounds of the general formula (1) 'Structure 1' indicated above can be prepared from tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, by reaction with compounds of the general formula (3) 'Structure 3' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al.

When X represents O, the compounds of the general formula (1) 'Structure 1' indicated above can be prepared from metal salts or organic base salts of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, by reaction with compounds of the general formula (4) 'Structure 4'. 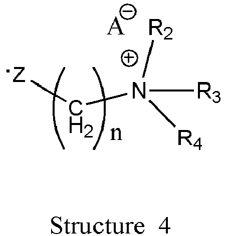 In structure 4, R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; Z represents halogen, or p-toluenesulphonyl, A⁻ represents Cl⁻, Br⁻, F, I⁻, AcO⁻, citrate, or any negative ions; and n=0,1,2,3,4,5.....

When X represents O, the compounds of the general formula (1) 'Structure 1' indicated above can be prepared from immobilized base salts of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds of the general formula (5) 'Structure 5', 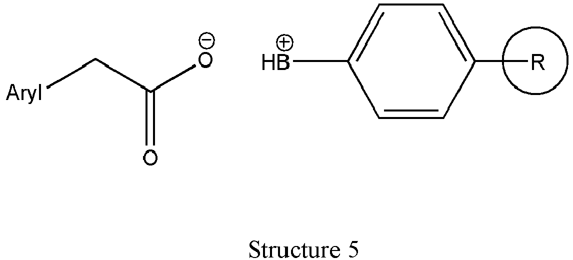 In structure 5, R represents cross-linked resin; Aryl represents aryl- and heteroaryl groups that are listed in 'structure 1', B represents any base groups, such as pyridine, piperidine, triethylamine, or other base groups, by reaction with compounds of the general formula (4) 'Structure 4'.

### Advantageous Effects

These pro-drugs of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds have a lipophilic portion and a hydrophilic portion (the amine groups that exist in the protonated form at physiological pH). The positively charged amino groups of these pro-drugs have two major advantages. First, it largely increases the solubility of the drugs; when these new prodrugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drugs were changed back to the drugs itself. The pro-drugs have a much better absorption rate, and thus the pro-drugs will have better strength than tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds at the same dosage. The experiment results suggest that the pro-drugs, diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, diethylaminoethyl 4-(4-chlorophenyl)-2-phenyl-5-thiazoleacetate.AcOH, diethylaminoethyl 3-(4-chlorophenyl)-1-phenyl-1H-pyrazole-4-acetate.AcOH diffuses through human skin -100 times faster than do tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds. It takes 2-4 hours for tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds to reach the peak plasma level when they are taken orally, but these prodrugs only took about 40-50 minutes to reach the peak plasma level. The most exciting result is that the pro-drugs can be administered not only orally, but also transdermally for any type of medical treatment and should avoid most of the side effects of tolmetin, zomepirac, etodolac, amfenac, bromofenac, alclofenac, fenclofenac, acemetacin, indomethacin, sulindac, fentiazac, lonazolac, bendazac, and related compounds, most notably GI disturbances such as dyspepsia, gastroduodenal bleeding, gastric ulcerations, and gastritis. Another great benefit of transdermal administration of these pro-drugs is that administering medication, especially to children, will be much easier.

### Description of Drawings

Figure 1: Cumulative amounts of diethylaminoethyl 1-(p-chlorobenzoyl)-5-methoxy-2-methylindole 3-acetate.AcOH (A, 30% solution), diethylaminoethyl (Z)-5-fluoro-2-methyl-1-[(4-methylsulfinyl) phenylmethylene] - 1H-indene-3-acetate.AcOH (B, 30% solution), diethylaminoethyl 1-methyl-5-(4-methylbenzoyl)-1H-pyrrole-2-acetate.AcOH (C, 30% solution), diethylaminoethyl 5-(4-Chlorobenzoyl)-1,4-dimethyl-1H-pyrrole-2-acetate.AcOH (D, 30% solution), diethylaminoethyl 1,8-diethyl-1,3,4,9-tetrahydropyrano-[3,4-b] indole-1-acetate.AcOH (E, 30% solution), indomethacin (F, 30% suspension), sulindac (G, 30% suspension), tolmetin (H, 30% suspension), zomepirac (I, 30% suspension), or etodolac (J, 30% suspension). crossing isolated human skin tissue in Franz cells (n=5). In each case, the vehicle was pH 7.4 phosphate buffer (0.2 M).
Figure 2: Cumulative amounts of diethylaminoethyl 2-amino-3-benzoylbenzeneacetate.AcOH (A, 30% solution), diethylaminoethyl 2-amino-3-(4-bromo-benzoyl)benzeneacetate.AcOH (B, 30% solution), diethylaminoethyl 3-chloro-4-(2-propenyloxy)benzeneacetate.AcOH (C, 30% solution), diethylaminoethyl 2-(2,4-dichlorophenoxy) benzeneacetate.AcOH (D, 30% solution), diethylaminoethyl 1-(4-chlorobenzoyl-5-methoxy-2-methyl-1H-indole-3-acetoxyacetate.AcOH (E, 30% solution), diethylaminoethyl 4-(4-chlorophenyl)-2-phenyl-5-thiazoleacetate.AcOH (F, 30% solution), amfenac (G, 30% suspension), bromofenac (H, 30% suspension), alclofenac (I, 30% suspension), fenclofenac (J, 30% suspension), acemetacin (K, 30% suspension), or fentiazac (L, 30% suspension). crossing isolated human skin tissue in Franz cells (n=5). In each case, the vehicle was pH 7.4 phosphate buffer (0.2 M).
Figure 3: Total plasma levels of indomethacin, sulindac, tolmetin, or zomepirac after topical application of 1 ml of a 20% solution of diethylaminoethyl 1-(p-chlorobenzoyl)-5-methoxy-2-methylindole 3-acetate.AcOH (A), diethylaminoethyl (Z)-5-fluoro-2-methyl-1-[(4-methylsulfinyl) phenylmethylene] - 1H-indene-3-acetate.AcOH (B), diethylaminoethyl 1-methyl-5-(4-methylbenzoyl)-1H-pyrrole-2-acetate.AcOH (C), diethylaminoethyl 5-(4-Chlorobenzoyl)-1,4-dimethyl-1H-pyrrole-2-acetate.AcOH (D), indomethacin (E), sulindac (F), tolmetin (G), or zomepirac (H) in isopropanol to the backs of hairless mice (n=5).
Figure 4: Total plasma levels of etodolac, amfenac, bromofenac, or alclofenac after topical application of diethylaminoethyl 1,8-diethyl-1,3,4,9-tetrahydropyrano-[3,4-b] indole-1-acetate.AcOH (A), diethylaminoethyl 2-amino-3-benzoylbenzeneacetate.AcOH (B), diethylaminoethyl 2-amino-3-(4-bromo-benzoyl)benzeneacetate.AcOH (C), diethylaminoethyl 3-chloro-4-(2-propenyloxy) benzeneacetate.AcOH (D), etodolac (E), amfenac (F), bromofenac (G), or alclofenac (H) 1 ml of a 20% solution of in isopropanol to the backs of hairless mice (n=5).
Figure 5: Total plasma levels of fenclofenac, acemetacin, fentiazac, or lonazolac after topical application of diethylaminoethyl 2-(2,4-dichlorophenoxy)benzeneacetate.AcOH (A), diethylaminoethyl 1-(4-chlorobenzoyl-5-methoxy-2-methyl-1H-indole-3-acetoxyacetate.AcOH (B), diethylaminoethyl 4-(4-chlorophenyl)-2-phenyl-5-thiazoleacetate.AcOH (C), diethylaminoethyl 3-(4-chlorophenyl)-1-phenyl-1H-pyrazole-4-acetate.AcOH (D), fenclofenac (E), acemetacin (F), fentiazac (G), or lonazolac )H) 1 ml of a 20% solution of in isopropanol to the backs of hairless mice (n=5).
Figure 6: The prolongation time of the pain threshold of mice tails after 50mg/kg of diethylaminoethyl 1-(p-chlorobenzoyl)-5-methoxy-2-methylindole 3-acetate.AcOH (B), diethylaminoethyl (Z)-5-fluoro-2-methyl-1-[(4-methylsulfinyl) phenylmethylene] - 1H-indene-3-acetate.AcOH (C), diethylaminoethyl 1-methyl-5-(4-methylbenzoyl)-1H-pyrrole-2-acetate.AcOH (D), diethylaminoethyl 5-(4-Chlorobenzoyl)-1,4-dimethyl-1H-pyrrole-2-acetate.AcOH (E), diethylaminoethyl 1,8-diethyl-1,3,4,9-tetrahydropyrano-[3,4-b]indole-1-acetate.AcOH (F), diethylaminoethyl 2-amino-3-benzoylbenzeneacetate.AcOH (G) were administered transdermally. A group is the control group.
Figure 7: The prolongation time of the pain threshold of mice tails after 50mg/kg of diethylaminoethyl 2-amino-3-(4-bromo-benzoyl)benzeneacetate.AcOH (B), diethylaminoethyl 3-chloro-4-(2-propenyloxy)benzeneacetate.AcOH (C), diethylaminoethyl 2-(2,4-dichlorophenoxy)benzeneacetate.AcOH (D), diethylaminoethyl 1-(4-chlorobenzoyl-5-methoxy-2-methyl-1H-indole-3-acetoxyacetate.AcOH (E), diethylaminoethyl 4-(4-chlorophenyl)-2-phenyl-5-thiazoleacetate.AcOH (F), diethylaminoethyl 3-(4-chlorophenyl)-1-phenyl-1H-pyrazole-4-acetate.AcOH (G). were administered transdermally. A group is the control group.
Figure 8. The rate of swelling (%) after a carrageenin injection. 1 hour before the carrageenin injection, diethylaminoethyl 1-(p-chlorobenzoyl)-5-methoxy-2-methylindole 3-acetate.AcOH (100 mg/kg, B), diethylaminoethyl (Z)-5-fluoro-2-methyl-1-[(4-methylsulfinyl) phenylmethylene] - 1H-indene-3-acetate.AcOH (100 mg/kg, C), diethylaminoethyl 1-methyl-5-(4-methylbenzoyl)-1H-pyrrole-2-acetate.AcOH (100 mg/kg, D), diethylaminoethyl 5-(4-Chlorobenzoyl)-1,4-dimethyl-1H-pyrrole-2-acetate.AcOH (100 mg/ kg, E), diethylaminoethyl 1,8-diethyl-1,3,4,9-tetrahydropyrano-[3,4-b] indole-1-acetate.AcOH (100 mg/kg, F), diethylaminoethyl 2-amino-3-benzoylbenzeneacetate.AcOH (100 mg/kg, G) were administered transdermally. A group is the control group.
Figure 9. The rate of swelling (%) after a carrageenin injection. 1 hour before the carrageenin injection, diethylaminoethyl 2-amino-3-(4-bromo-benzoyl)benzeneacetate.AcOH (100 mg/kg, H), diethylaminoethyl 3-chloro-4-(2-propenyloxy)benzeneacetate.AcOH (100 mg/kg, I), diethylaminoethyl 2-(2,4-dichlorophenoxy)benzeneacetate.AcOH (100 mg/kg, J), diethylaminoethyl 1-(4-chlorobenzoyl-5-methoxy-2-methyl-1H-indole-3-acetoxyacetate.AcOH (100 mg/ kg, K), diethylaminoethyl 4-(4-chlorophenyl)-2-phenyl-5-thiazoleacetate.AcOH (100 mg/kg, L), or diethylaminoethyl 3-(4-chlorophenyl)-1-phenyl-1H-pyrazole-4-acetate.AcOH (100 mg/kg, M) were administered transdermally. A is the control group.

Structure 1. In structure 1, Aryl represents aryl- and heteroaryl groups according to Claim 1 , R represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S, NH, OCH₂ COO, OCH₂ COS, or OCH₂CONH; A⁻ represents Cl⁻, Br⁻, F, I⁻, AcO⁻, citrate, or any negative ions; and n=0,1,2,3,4,5,6,7,8,9,10 .......

### Best Mode

### Preparation of diethylaminoethyl (Z)-5-fluoro-2-methyl-l-[(4-methylsulfinyl) phenylmethylene] - 1H-indene-3-acetate.AcOH

11.7 g (0.1 mol) of diethylaminoethanol was dissolved in 10% sodium bicarbonate (200 ml) and acetone (100 ml). 37.5 g (0.1 mol) of (Z)-5-fluoro-2-methyl-1-[(4-methylsulfinyl) phenylmethylene]-1H-indene-3-acetyl chloride was added into the reaction mixture. The mixture is stirred for 3 hours at RT. The solvents are evaporated off. The residue is suspended in ethyl acetate (500ml). 5% sodium bicarbonate (200 ml) is added into the reaction mixture with stirring. Ethyl acetate layer is collected and washed with water (3 x 500 ml). The ethyl acetate solution was dried over anhydrous sodium sulfate. Sodium sulfate is removed by filtration. 6 g of acetic acid is added into the reaction mixture with stirring. The organic solution was evaporated off. After drying, it yielded 42 g of the desired product (81.5 %). Hygroscopic product; Solubility in water: 400 mg/ml; Elementary analysis: C₂₈H₃₄ FNO₅S; MW: 515.64. Calculated % C: 65.22; H: 6.65; F: 3.68; N: 2.72; O: 15.51; S: 6.22; Found % C: 65.20; H: 6.67; N: 3.67; O: 15.53; S: 6.21. ¹H-NMR (400 MHz, D₂ O): δ: δ: 1.36 (t, 6H), 1.65 (s, 3H), 2.11 (s, 3H), 2.56 (s, 3H), 2.82 (s, 2H), 3.27 (m, 4H), 3.47(m, 2H), 4.48 (t, 2H), 6.74 (s, 1H), 6.84 (m,1H), 6.88 (b, 1H), 7.00 (m, 1H), 7.30 (m, 1H), 7.63 (m, 2H), 7.65 (m, 2H).

### Mode for Invention

### Preparation of diethylaminoethyl 1-methyl-5-(4-methylbenzoyl)-1H-pyrrole-2-acetate.AcOH.

28.6 g (0.1 mol) of 1-methyl-5-(4-methylbenzoyl)-1H-pyrrole-2-acetyl chloride was dissolved in 100 ml of chloroform. The mixture was cooled to 0°C. 15 ml of triethylamine and 8.9 g (0.1 mol) of dimethylaminoethanol were added into the reaction mixture. The mixture is stirred for 3 hours at RT. The solvents are evaporated off. The residue is dissolved in methanol (300ml), 5% sodium bicarbonate (200 ml) is added into the reaction mixture. The mixture is stirred for 3 hr. The mixture is evaporated to dryness. Methanol (300 ml) is added into the residue with stirring. Solid is removed by filtration and washed with methanol. The solution is evaporated to dryness and the residue is dissolved in chloroform (200 ml). 6 g of acetic acid is added into the reaction mixture with stirring. Some solid is removed by filtration. Another 6 g of acetic acid is added into the reaction mixture with stirring. The organic solution was evaporated off. After drying, it yielded 37 g of the desired product (88.8%). Hygroscopic product; Solubility in water: 500 mg/ml; Elementary analysis: C₂₃H₃₂N₂O₅; MW: 416.51. Calculated % C: 66.32; H: 7.74; N: 6.73; O: 19.21; Found % C: 66.29; H: 7.76; N: 6.73; O: 19.22. ¹H-NMR (400 MHz, D₂O): δ: 1.51 (t, 6H), 2.21 (s, 3H), 2.35 (s, 3H), 3.25 (m, 4H), 3.48 (s, 3H), 3.50 (m, 2H), 3.55 (s, 2H), 4.50 (t, 2H), 5.86 (m, 1H), 6.67 (m, 1H), 6.70 (b, 1H), 7.25 (m, 2H), 7.67 (m, 2H).

### Preparation of S-dimethylaminoethyl 1-(p-chlorobenzoyl)-5-methoxy-2-methylindole 3-acetate.AcOH.

10.4 g (0.1 mol) of dimethylaminoethyl mercaptan was dissolved in 10% sodium bicarbonate (200 ml) and acetone (100 ml). 37.6 g (0.1 mol) of 1-(p-chlorobenzoyl)-5-methoxy-2-methylindole 3-acetyl chloride was added into the reaction mixture. The mixture is stirred for 3 hours at RT. The solvents are evaporated off. The residue is suspended in ethyl acetate (500ml). 5% sodium bicarbonate (200 ml) is added into the reaction mixture with stirring. Ethyl acetate layer is collected and washed with water (3 x 500 ml). The ethyl acetate solution was dried over anhydrous sodium sulfate. Sodium sulfate is removed by filtration. 6 g of acetic acid is added into the reaction mixture with stirring. The organic solution was evaporated off. After drying, it yielded 46 g of the desired product (86.3%). Hygroscopic product; Solubility in water: 400 mg/ml; Elementary analysis: C₂₇H₃₃ClN₂O₅S; MW: 533.08. Calculated % C: 60.83; H: 6.24; Cl: 6.65; N: 5.26; O: 15.01; S: 6.02. Found % C: 60.80; H: 6.26; Cl: 6.66; N: 5.25, O: 15.02; S: 6.01. ¹H-NMR (400 MHz, D₂O): δ: 1.47 (t, 6H), 2.20 (s, 3H), 2.28 (s, 3H), 3.28 (m, 4H), 3.31(t, 2H), 3.54 (s, 2H), 3.73 (s, 3H), 3.91 (t, 2H), 6.70 (b, 1H), 6.24 (m, 1H), 6.68 (m, 1H), 6.75 (m, 1H), 7.46 (m, 2H), 7.75 (m, 2H).

### Preparation of N-dimethylaminoethyl 5-(4-Chlorobenzoyl)-1,4-dimethyl-1H-pyrrole-2-acetamide.AcOH

8.8 g (0.1 mol) of dimethylaminoethylamine was dissolved in 10% sodium bicarbonate (200 ml) and acetone (100 ml). 31 g (0.1 mol) of 2-(3-benzoyphenyl) propionyl chloride was added into the reaction mixture. The mixture is stirred for 3 hours at RT. The solvents are evaporated off. The residue is suspended in ethyl acetate (500ml). 5% sodium bicarbonate (200 ml) is added into the reaction mixture with stirring. Ethyl acetate layer is collected and washed with water (3 x 500 ml). The ethyl acetate solution was dried over anhydrous sodium sulfate. Sodium sulfate is removed by filtration. 6 g of acetic acid is added into the reaction mixture with stirring. The organic solution was evaporated off. After drying, it yielded 33 g of the desired product (85.9 %). Hygroscopic product; Solubility in water: 400 mg/ml; Elementary analysis: C₂₃H₃₂ClN₃O₄; MW: 449.97. Calculated % C: 61.39; H: 7.17; Cl: 7.88; N: 9.34; O: 14.22; Found % C: 61.37; H: 7.18; Cl: 7.89; N: 9.32; O: 14.24. ¹H-NMR (400 MHz, D₂O): δ: 1.42 (t, 6H), 2.05 (s, 3H), 2.21 (s, 3H), 3.26(m, 4H), 3.48 (s, 2H), 3.50 (t, 2H), 3.64 (t, 2H), 3.50 (s, 3H), 5.66 (s, 1H), 7.0 (b, 1H), 7.46 (m, 2H), 7.54 (m, 2H), 7.47 (b, 1H).

### Preparation of N-dimethylaminoethyl 1,8-diethyl-1,3,4,9-tetrahydropyrano-[3,4-blindole-1-aeetamide.AcOH

28.9 g (0.1 mol) of 1,8-diethyl-1,3,4,9-tetrahydropyrano-[3,4-b]indole-1-acetic acid was dissolved in 100 ml of acetonitrile. 32.1 g of O-(Benzotriazol-1-yl)-N,N,N',N'-tetramethyluronium tetrafluoroborate and 30 ml of triethylamine were added into the reaction mixture. 11.7 g of dimethylaminoethylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 40 g of the desired product (89.4%). Hygroscopic product; Solubility in water: 400 mg/ml; Elementary analysis: C₂₅H₄₁N₃O₄; MW: 447.61. Calculated % C: 67.08; H: 9.23; N: 9.39; O: 14.30; Found % C: 67.05; H: 9.25; N: 9.38; O: 14.32. ¹H-NMR (400 MHz, D₂ O): δ: 0.95 (t, 3H), 1.20 (t, 3H), 1.24 (t, 3H), 1.40 (m, 2H), 1.50 (s, 6H), 1.87 (m, 2H), 2.21 (s, 3H), 2.25 (s, 2H), 2.56 (m, 2H), 3.08 (m, 1H), 3.11 (m, 1H), 3.28 (m, 4H), 3.50 (m, 2H), 3.64 (m, 2H), 7.0 (b, 1H), 6.43 (m, 1H), 6.70 (m, 1H), 6.72 (m, 1H), 7.6 (b, 1H).

### Preparation of diethylaminoethyl 3-chloro-4-(2-propenyloxy) benzeneacetate.AcOH

60 g of Polymer-bound triethylamine (3 mol/g, 100-200 mesh) was suspended in 180 ml of chloroform. 22.7 g (0.1 mol) of 3-chloro-4-(2-propenyloxy) benzeneacetic acid was added into the mixture with stirring. 43 g (0.15mol) of diethylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 34 g of the desired product (88.3%). Hygroscopic product; Solubility in water: 400 mg/ml; Elementary analysis: C₁₉H₂₉ClN₂O₄; MW: 384.9. Calculated % C: 59.29; H: 7.59; Cl: 9.21, N: 7.28; O: 16.63; Found % C: 59.26; H: 7.61; Cl: 9.22; N: 7.26; O: 16.65.1H-NMR (400 MHz, D₂O: δ: 1.56 (t, 6H), 2.21 (s, 3H), 3.27 (m, 4H), 3.43 (s, 2H), 3.50 (m, 2H), 3.63 (m, 2H), 4.60 (s, 2H), 5.23 (m, 2H), 5.89 (m, 1H), 6.5 (b, 1H), 6.61 (m, 1H), 6.81 (m, 1H), 6.94 (m, 1H), 7.70 (b, 1H).

### Industrial Applicability

The pro-drugs of the general formula (1) 'Structure 1' are superior to tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds. They may be used medicinally in treating any tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds-treatable conditions in humans or animals. They may be used for the relief of signs and symptoms of rheumatoid arthritis and osteoarthritis, the reduction of fever, and the treatment of dysmenorrhea. They may be also prescribed for diabetic neuropathy and acute migraine headache. Due to their very high membrane penetration rate, these prodrugs can be used in treating asthma by inhalation to a host. They can be used to treat acne due to their anti-inflammatory properties.

**Sequence List Text**

## Claims

1. The compounds of the general formula (1) 'Structure 1' 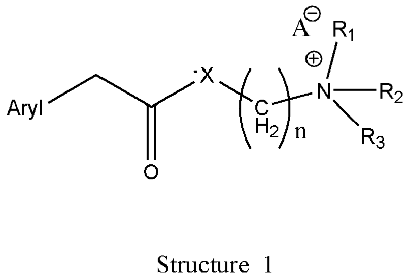 In structure 1, R₁ represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S, NH, OCH₂COO, OCH₂COS, or OCH₂CONH; A⁻ represents Cl⁻, Br⁻, F⁻, I⁻, AcO⁻, citrate, or any negative ions; and n=0,1,2,3,4,5,6,7,8,9,10 ......; Aryl represents 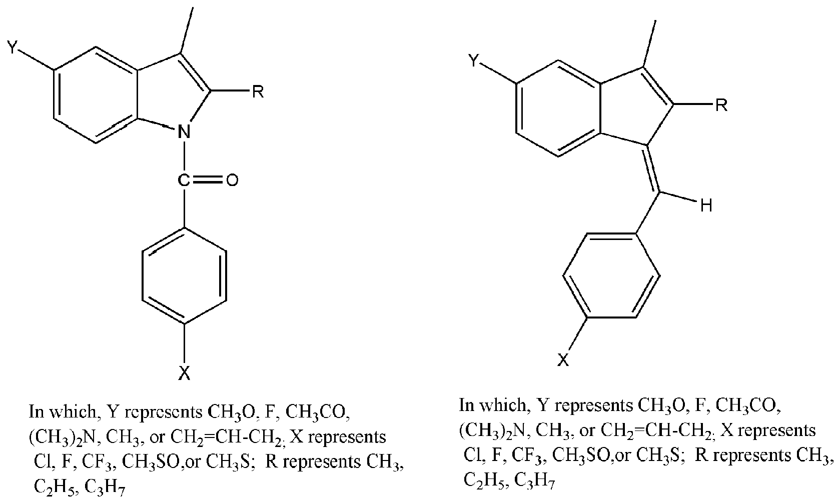 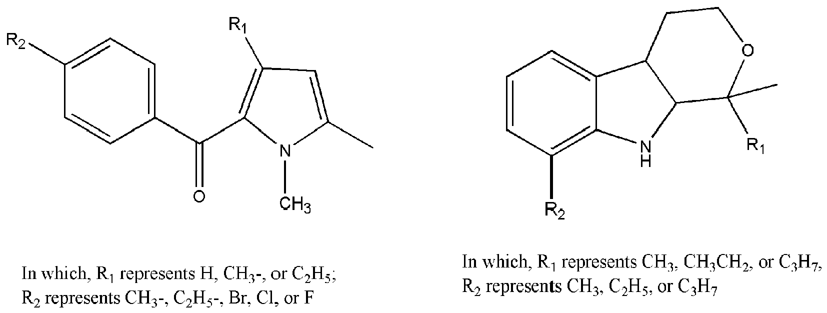 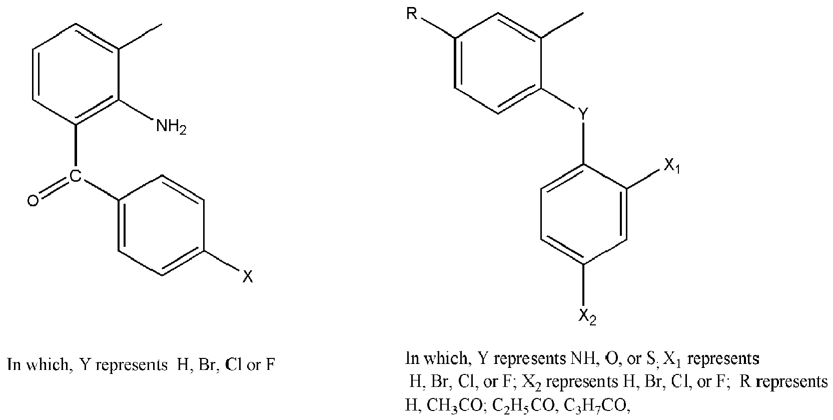 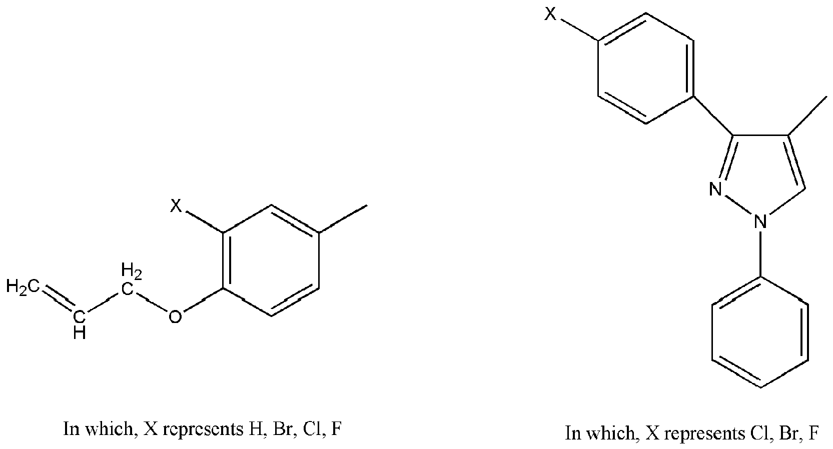 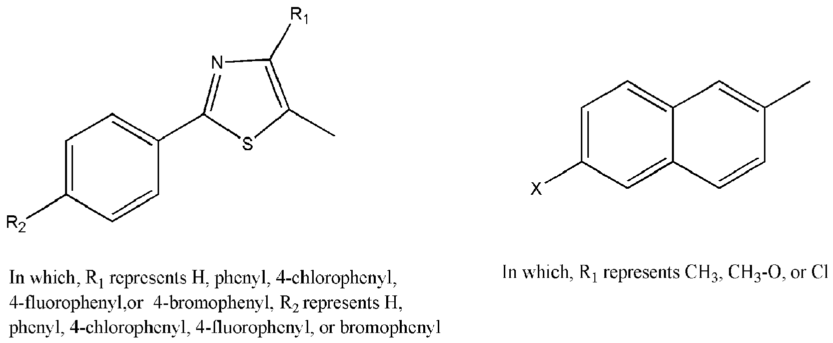 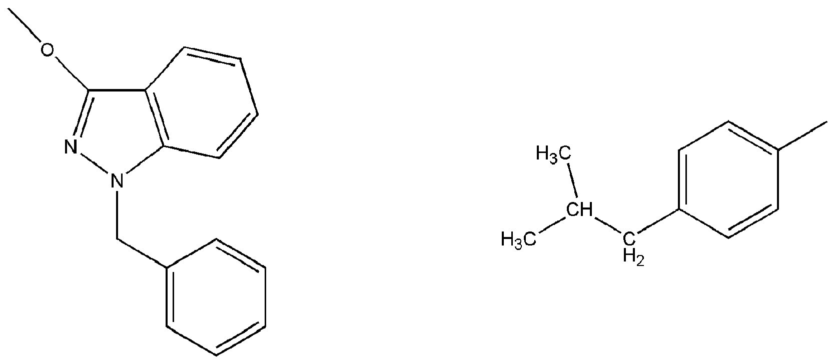 , all R groups may include C, H, O, S, N atoms and may have single, double, and treble bonds. Any CH₂ groups may be replaced with O, S, or NH.

2. Processes for the preparation of compounds of the general formula (1) 'Structure 1' according to Claim 1, wherein functional derivatives of tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds , for example, acid halides or mixed anhydrides are reacted with compounds of the general formula (3) 'Structure 3', 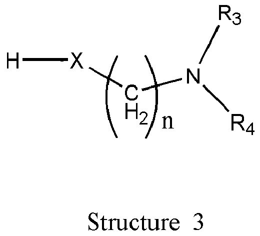 In structure 3, R₃ represents H, one of any alkyl, alkyloxy, alkenyl, or alkynyl residues having 1 to 12 carbon atoms, or aryl residues; R 4 represents H, one of any alkyl, alkyloxy, alkenyl, or alkynyl residues having 1 to 12 carbon atoms, or aryl residues; X represents O, S or NH; and n=0,1,2,3,4,5,6,7,8,9,10......

3. Processes for the preparation of the compounds of the general formula (1) 'Structure 1' according to Claim 1, wherein tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds are reacted with compounds of the general formula (3) 'Structure 3' according to Claim 3, by using coupling reagents, such as N,N'-Dicyclohexylcarbodiimide, N, N'-Diisopropylcarbodiimide, O-(Benzotriazol-l-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al.

4. Processes for the preparation of compounds of the general formula (1) 'Structure 1' according to Claim 1. wherein metal salts, organic base salts or immobilized base salts of tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds are reacted with compounds of the general formula (4) 'Structure 4', 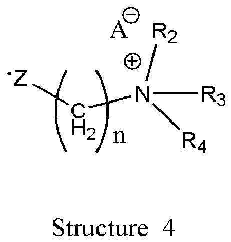 In structure 4, R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; R₄ represents H, one of any alkyl, alkyloxy, alkenyl, or alkynyl residues having 1 to 12 carbon atoms, or aryl residues; Z represents halogen, or p-toluenesulphonyl, A⁻ represents Cl⁻, Br⁻, F⁻, I⁻, AcO⁻, citrate, or any negative ions; and n=0,1,2,3,4,5.....

5. Compounds of the general 'Structure 1' or a composition comprising of at least one compound of the general formula (1) 'Structure 1', as an active ingredient, according to Claim 1, where they can be administered orally or transdermally, for treating any NSAIAs-treatable conditions in humans or animals. The NSAIAs-treatable conditions include, but are not limited to, pain from a toothache, headache, and arthritis and other inflammatory pain, fever, cancer, dysmenorrhea, radiation-induced vomiting, diabetic neuropathy and acute migraine headache, hemophilic arthropathy, bone loss, and sunburn.

6. Methods for treating any NSAIAs-treatable conditions in humans or animals by administering transdermally to any part of body (in the from of a solution, spray, lotion, ointment, emulsion or gel) to deliver therapeutically effective plasma levels of compounds of the general formula (1) 'Structure 1' according to Claim 1.

7. Methods for topically treating pain such as a headache, toothache, and muscle pain, and arthritis and other inflammatory pain in humans or animals by administering to the inflamed area a therapeutically effective amount of the compounds of the general formula (1) 'Structure 1' or a composition comprising at least one compound of the general formula (1) 'Structure 1', as an active ingredient, according to Claim 1.

8. Compounds of the general formula (1) 'Structure 1' or a composition comprising of at least one compound of the general formula (1) 'Structure 1', as an active ingredient, according to Claim 1, may be administered transdermally, for treating acne, sunburn or other skin disorders in the from of a solution, spray, lotion, ointment, emulsion or gel.

9. Compounds of the general 'Structure 1' or a composition comprising of at least one compound of the general formula (1) 'Structure 1', as an active ingredient, according to Claim 1, are administered by spraying to through the mouth or nose or other parts of body for treating asthma.

10. Compounds of the general 'Structure 1' or a composition comprising of at least one compound of the general formula (1) 'Structure 1', as an active ingredient, according to Claim 1, for treating any eye inflammatory diseases, for treating of ocular pain after corneal surgery, for treating glaucoma or for treating ear inflammatory and/or painful conditions (otitis) in humans or animals.

11. Transdermal therapeutic application systems of Compounds of the general formula (1) 'Structure 1' or a composition comprising of at least one compound of the general formula(1) 'Structure 1', as an active ingredient, according to claim 1 for treating any NSAIAs-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables the NSAIAs to reach constantly optimal therapeutic blood levels to increase effectiveness and reduce the side effects of NSAIAs.
